(19) Europäisches Patentamt
European Patent Office
Office européen des brevets

(11) EP 0 534 823 B1

(12) # FASCICULE DE BREVET EUROPEEN

(45) Date de publication et mention de la délivrance du brevet:
**24.07.1996 Bulletin 1996/30**

(51) Int. Cl.$^6$: **A61K 7/00**, A61K 7/02, A61K 7/48

(21) Numéro de dépôt: **92402546.3**

(22) Date de dépôt: **17.09.1992**

(54) **Composition cosmétique anhydre de maquillage comprenant une phase grasse et procédé de traitement cosmétique utilisant cette composition**

Fettphase enthaltende wasserfreie kosmetische Schminke sowie kosmetisches Behandlungsverfahren unter Verwendung dieses Mittel

Anhydrous make-up composition containing a fatty phase and the process for cosmetic use of this composition

(84) Etats contractants désignés:
**AT BE CH DE DK ES FR GB GR IT LI NL PT SE**

(30) Priorité: **30.09.1991 FR 9111985**

(43) Date de publication de la demande:
**31.03.1993 Bulletin 1993/13**

(73) Titulaire: **L'OREAL**
**F-75008 Paris (FR)**

(72) Inventeurs:
- **Ribier, Alain**
  **F-75014 Paris (FR)**
- **Simmonet, Jean-Thierry**
  **F-75011 Paris (FR)**
- **Miguel, Dolorès**
  **F-92340 Bourg la Reine (FR)**

(74) Mandataire: **Peuscet, Jacques et al**
**SCP Cabinet Peuscet et Autres,**
**68, rue d'Hauteville**
**F-75010 Paris (FR)**

(56) Documents cités:
**EP-A- 0 087 993**
**EP-A- 0 120 722**
**EP-A- 0 158 441**
**FR-A- 2 390 159**
**LU-A- 54 005**

- **PATENT ABSTRACTS OF JAPAN, vol. 4, no. 73 (C-12)(555), 28 mai 1980; & JP-A-55 38 332 (KANEBO K.K.) 17-03-1980**

EP 0 534 823 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.13.0/3.4

## Description

La présente invention concerne des compositions cosmétiques anhydres de maquillage comprenant une phase grasse et un procédé de traitement cosmétique utilisant ces compositions.

Les compositions cosmétiques anhydres de façon connue comprennent une phase grasse contenant des huiles minérales ou organiques, des corps gras, des tensioactifs destinés à l'obtention d'une phase homogène et, le plus souvent, des cires. Des colorants et pigments sont ajoutés quand on désire que la composition anhydre soit colorée.

Les compositions cosmétiques anhydres de maquillage telles que les rouges à lèvres, les fards à paupière, les fonds de teint coulés, les fards à joues coulés ont pour principale fonction de permettre l'application de colorants et pigments sur la peau, de façon régulière, homogène et durable.

Il est par ailleurs connu d'utiliser des compositions cosmétiques et/ou dermopharmaceutiques qui ont pour fonction de traiter la peau par application topique.

Les exigences de la vie moderne font que l'on cherche à simplifier les traitements cosmétiques en utilisant des compositions cosmétiques ayant plusieurs fonctions de façon à réduire le nombre des opérations de traitement cosmétique nécessaires.

On a donc cherché à introduire dans les compositions anhydres de maquillage des actifs cosmétiques et/ou dermopharmaceutiques. Dans ce but, on a essayé d'introduire des actifs lipophiles qui peuvent être solubilisés dans la phase grasse des compositions anhydres. Cette phase grasse contenant souvent une grande quantité de cire, son absorption par la peau ou la muqueuse sera très limitée. Par conséquent, l'efficacité des actifs, qui est fonction de la pénétration de la phase grasse dans la peau, sera également limitée.

On a également pensé à introduire des actifs hydrosolubles. Mais leur introduction nécessite des mesures technologiques coûteuses telles que la lyophilisation des actifs puis leur microdispersion dans la phase grasse ou l'incorporation des actifs dans des supports solides tels que des microsphères ou des microcapsules puis leur dispersion dans la phase grasse. Dans ce dernier cas, la quantité d'actif que l'on peut introduire est limitée car l'incorporation de supports solides contenant les actifs modifie de façon importante les propriétés mécaniques des compositions anhydres.

On peut donc considérer que jusqu'à présent le problème de l'introduction d'actifs cosmétiques et/ou dermopharmaceutiques dans des compositions cosmétiques anhydres n'a pas été résolu de façon satisfaisante.

Ce problème se pose également dans le cas de produits cosmétiques anhydres tels que les sticks déodorants et les anticernes anhydres.

D'autre part, il est connu que certains lipides amphiphiles ioniques ou non-ioniques et que certains mélanges de lipides amphiphiles sont susceptibles de former, par contact avec une phase aqueuse, des vésicules constituées de feuillets plus ou moins sphériques de phase lipidique vésiculaire encapsulant la phase aqueuse.

De façon connue des additifs peuvent être introduits dans la phase lipidique vésiculaire pour améliorer la stabilité et la perméabilité des vésicules obtenues. Ces additifs peuvent être des stérols, en particulier le cholestérol ou le dicétylphosphate.

Dans la suite de la demande et dans les revendications, le terme "phase lipidique provésiculaire" désignera des lipides amphiles susceptibles de former des vésicules, des mélanges de lipides amphiphiles ioniques et/ou non-ioniques susceptibles de former des vésicules et des lipides amphiphiles ou mélanges de lipides amphiphiles contenant des additifs pour améliorer la stabilité et la perméabilité.

Il est bien connu que ces vésicules ont déjà par elles-mêmes une action cosmétique par application topique mais surtout qu'elles permettent l'encapsulation d'actifs cosmétiques et/ou dermopharmaceutiques hydrosolubles et liposolubles dans la phase lipidique et/ou la phase aqueuse. La préparation de vésicules de lipides amphiphiles et leur utilisation en cosmétique sont, par exemple, décrites dans FR-A 2 315 991.

On sait par ailleurs par FR-A 2416 008, que les vésicules lipidiques peuvent être lyophilisées et qu'après lyophilisation ces vésicules conservent leurs propriétés traitantes. On pourrait donc penser à introduire des liposomes et/ou des niosomes lyophilisés dans des compositions cosmétiques anhydres de maquillage. Mais cette opération est compliquée et coûteuse au plan industriel, car elle nécessite de fabriquer des vésicules de lipide(s) amphiphile(s), de les lyophiliser en présence d'agents antimottants et/ou cryoprotecteurs sans intérêt spécifique pour des compositions cosmétiques, puis de les incorporer à haute température dans un produit cosmétique complexe comportant des huiles fondues et des cires.

Selon la présente invention, la demanderesse a trouvé que la phase lipidique provésiculaire susceptible de former des vésicules en présence d'eau conservait cette propriété de former des vésicules, lorsqu'elle était mélangée à une phase grasse, généralement utilisée pour la fabrication de compositions cosmétiques anhydres qui contient des huiles organiques et/ou minérales, des corps gras,le plus souvent des cires, et des tensioactifs.

En effet, comme montré dans les exemples comparatifs donnés ci-après, on observe après contact avec l'eau, la formation au sein de la phase aqueuse, de vésicules de qualité satisfaisante et en quantité importante. De plus, on constate que les vésicules formées conservent leur capacité d'encapsuler des actifs hydrophile et/ou lipophile et que dans ces conditions l'efficacité d'actifs introduits dans la composition anhydre est très nettement améliorée.

Il n'était pas évident pour l'Homme de l'Art que lorsque la phase lipidique provésiculaire susceptible de former des vésicules en présence d'eau était mélangée à la phase grasse utilisée pour la fabrication de cosmétiques de maquillage anhydre elle serait encore capable, en présence d'une phase aqueuse, par simple contact avec celle-ci de former des vésicules au sein de cette phase aqueuse. En particulier, on pouvait craindre que, lorsque la phase lipidique provésiculaire est constituée d'un mélange de lipide(s) amphiphile(s) ionique(s) et/ou de lipide(s) amphiphile(s) non-ionique(s) associés ou de lipide(s) amphiphile(s) associés à un ou plusieurs additifs, l'association de ces différents composés, qui peut être nécessaire pour obtenir la formation des vésicules sous une forme stable, ne soit détruite par les huiles, corps gras, cires, et les tensioactifs contenus dans la phase grasse du produit cosmétique anhydre.

Selon la présente invention on applique cette découverte à la fabrication de compositions cosmétiques anhydres contenant dans la phase grasse une phase lipidique provésiculaire susceptible de former des vésicules au contact de l'eau, ces compositions cosmétiques anhydres devant être mises en contact avec une phase aqueuse lors de leur utilisation pour provoquer la formation des vésicules.

Il faut noter que le contact avec une phase aqueuse est le plus souvent inhérent au mode d'application de ces compositions cosmétiques anhydres.

Par exemple, les rouges à lèvre sont mis en contact avec la muqueuse labiale, qui est riche en eau, et qui est régulièrement mouillée par la salive ; les fonds de teint coulés, les fards à paupière ou les fards à joue sont souvent hydratés juste avant application, à l'aide d'une éponge humide ; et les sticks déodorants sont appliqués sur une peau qui est humidifiée par les sécrétions sudorales.

La présente invention a donc pour premier objet un procédé de traitement cosmétique selon la revendication 1.

La présente invention a pour second objet les compositions cosmétiques selon la revendication 18.

De préférence la composition cosmétique contient un actif cosmétique et/ou dermopharmaceutique hydrosoluble et/ou liposoluble.

La quantité de phase lipidique provésiculaire contenant des lipides amphiphiles peut représenter 0,1 à 30% en poids de la composition cosmétique anhydre, de préférence de 1 à 20%.

Dans la phase lipidique provésiculaire, les lipides amphiphiles utilisés peuvent être tout lipide amphiphile connu pour la fabrication de vésicules en présence d'eau. Ces lipides sont, de façon connue, des lipides amphiphiles d'origine naturelle ou synthétique, ioniques ou non-ioniques, comportant, par molécule, une ou plusieurs longue(s) chaîne(s) hydrocarbonée(s) saturée(s) ou insaturée(s), linéaire(s) ou ramifiée(s), ayant de préférence 8 à 30 atomes de carbone, ces chaînes étant par exemple une chaîne oléique, lanolique, tétradécylique, hexadécylique, isostéarylique, laurique ou alcoylphényle, et un ou plusieurs groupement(s) hydrophile(s) pris parmi les groupes hydroxyle, étheroxyde, carboxyle, phosphate et amine.

Parmi les lipides amphiphiles ioniques, on préfère utiliser les phospholipides naturels (par exemple la lécithine d'oeuf ou de soja ou la sphingomyéline), les phospholipides de synthèse (par exemple, la dipalmitoyl-phosphatidylcholine ou la lécithine hydrogénée). On peut aussi utiliser des lipides amphotères comportant deux chaînes lipophiles ou une association de deux ions organiques à longue chaîne de signes opposés, ainsi que des lipides anioniques.

Parmi les lipides anioniques, on citera ceux qui sont décrits dans la demande de brevet LU 85 971 déposée le 23 juin 1985 et qui sont représentés par la formule :

$$\begin{array}{l} R_1\text{-CHOH-}\underset{\displaystyle |}{\text{CH}}\text{-COOM} \\ \quad\; R_2\text{-CONH} \end{array} \qquad (I)$$

formule dans laquelle :

$R_1$ désigne un radical alkyle ou alcényle en $C_7$ -$C_{21}$;

$R_2$ désigne un radical hydrocarboné, saturé ou insaturé, en $C_7$ -$C_{31}$; et

M représente H,Na,K,$NH_4$ ou un ion ammonium substitué dérivé d'une amine et, notamment d'une amine hydroxylée.

Pour les lipides amphiphiles non-ioniques, on préfère ceux contenant comme groupes hydrophiles des groupes polyoxyéthylénés ou polyglycérolés, ou des groupes dérivant d'esters de polyols oxyéthylénés ou non, ou encore des dérivés d'hydroxyamides. Avantageusement les lipides amphiphiles non-ioniques sont choisis dans le groupe formé par :

- (1) les dérivés de polyglycérol, linéaires ou ramifiés, de formule :

$$R_3O-\left[C_3H_5\ (OH)\ O\right]_{\bar{n}}-H \qquad (II)$$

formule dans laquelle :
- $C_3$ $H_5$ (OH) O- est représenté par les structures suivantes prises en mélange ou séparément :
$-CH_2-CHOH-CH_2O-$;

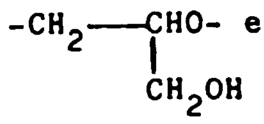 et 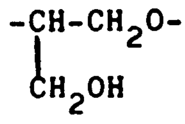

- $\bar{n}$ est une valeur statistique moyenne comprise entre 1 et 6.
$R_3$ représente :

(a) une chaîne atiphatique, linéaire ou ramifiée, saturée ou insaturée, contenant de 12 à 30 atomes de carbone ; ou des radicaux hydrocarbonés des alcools de lanoline ,
(b) un reste $R_4$ CO, où $R_4$ est un radical aliphatique, linéaire ou ramifié, en $C_{11}$ -$C_{29}$ ;
(c) un reste

$$R_5[OC_2H_3\ (R_6)]$$

où
$R_5$ peut prendre la signification (a) ou (b) donnée pour $R_3$ ; $-OC_2$ $H_3$ $(R_6)$- est représenté par les structures suivantes, prises en mélange ou séparément :

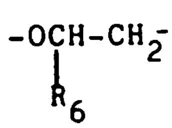 ; et 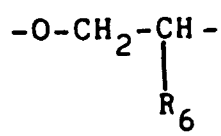

où $R_6$ prend la signification (a) donnée ci-dessus pour $R_3$
(2) - les éthers de polyglycérol, linéaires ou ramifiés, comportant deux chaînes grasses ;
(3) - les alcools gras polyoxyéthylénés ;
(4) - les stérols et phytostérols polyoxyéthylénés ;
(5) - les éthers de polyols ;
(6) - les esters de polyols oxyéthylénés ou non et, en particulier, les esters de sorbitol polyoxyéthylénés ;
(7) - les glycolipides d'origine naturelle ou synthétique, par exemple les cérébrosides ;
(8) - les alpha-diols polyglycérolés
(9) - les hydroxyamides représentés par la formule :

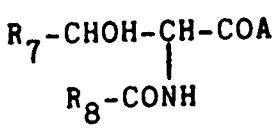 (III)

formule dans laquelle :

- $R_7$ représente un radical alkyle ou alcényle en $C_7$ -$C_{21}$ ;
- $R_8$ représente un radical hydrocarboné, saturé ou insaturé en $C_7$ - $C_{31}$ ;
- COA représente un groupement choisi parmi les deux groupements suivants :

. un reste

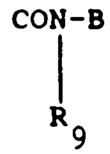

où :
B est un radical alkyle dérivé d'amines primaires ou secondaires, mono ou polyhydroxylées ; et
$R_9$ désigne un atome d'hydrogène ou un radical méthyle, éthyle ou hydroxyéthyle ; et

. un reste -COOZ, où Z représente le reste d'un polyol en $C_3$-$C_7$.

(10) les éthers et les esters décrits dans la demande du brevet français 90 14149 déposée le 14 Novembre 1990 par la demanderesse et ayant la formule :

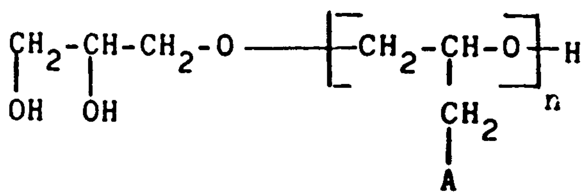
(IV)

formule dans laquelle A représente -OR, ou

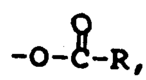

R représentant un radical hydrocarboné saturé ou insaturé et n représente une valeur égale à 2 ou une valeur statistique moyenne $\bar{n}$ supérieure à 1 et au plus égale à 6. R représente de préférence, un radical alkyle linéaire en $C_7$ -$C_{22}$, un radical alkyle ramifié en $C_7$ -$C_{36}$, un radical alkényle en $C_{18}$ ou un radical alkylaryle à chaîne alkyle linéaire ou ramifiée en $C_7$ -$C_{16}$;dans le radical alkylaryle, le groupe aryle est, de préférence, un groupe phényle ; le radical alkényle est, avantageusement un groupe octadécène-9 yle ou octadécanediène-9,12 yle.

De façon connue, divers additifs peuvent être associés aux lipides amphiphiles pour modifier leur stabilité et leur perméabilité. On citera, à cet égard, l'addition éventuelle des alcools et diols à longue chaîne ; des stérols comme par exemple le cholestérol et le B-sitostérol ; des amines à longue chaîne et leurs dérivés ammonium quaternaires ; des hydroxyalkylamines ; des amines grasses polyoxyéthylénées ; des esters d'aminoalcools à longue chaîne ; de leurs sels et dérivés ammonium quaternaires ; des esters phosphoriques d'alcools gras sous forme libre ou neutralisée comme par exemple le dicétylphosphate de sodium et des alkyl-sulfates comme par exemple le cétylsulfate de sodium ; des dérivés ioniques des stérols ; certains polymères, tels que les polypeptides et les protéines.

Comme expliqué ci-dessus, on peut, selon l'invention, introduire dans la phase grasse des actifs cosmétiques et/ou dermopharmaceutiques. On peut utiliser selon la présente invention tout actif connu pour avoir une action cosmétique et/ou dermopharmaceutique par application topique susceptible d'être encapsulé dans des vésicules de lipide amphiphile.

Parmi les actifs lipophiles, on choisira notamment dans le groupe formé par la vitamine E, les esters de vitamine E, les acides gras polyinsaturés, la vitamine F, les filtres solaires, les antioxydants, les conservateurs, la vitamine A, l'acide rétinoïque et ses esters.

Parmi les actifs hydrosolubles, on choisira de préférence ceux qui permettent par mélange à la phase lipidique provésiculaire l'obtention de phases lamellaires anhydres tels que le glycérol, le sorbitol et autres polyols de structure proche. On peut aussi choisir des acides aminés tels que l'arginine, la lysine, la proline, la sérine ; des vitamines telles que le D,L-panthénol ; et des filtres solaires.

De façon connue la phase grasse est constituée par des corps gras tels que le beurre de cacao et par des huiles. Parmi les huiles susceptibles d'être utilisées selon l'invention, on peut en particulier citer :

- les huiles minérales telles que l'huile de paraffine, l'huile de vaseline et les huiles ayant un point d'ébullition compris entre 310 et 410°C.
- les huiles d'origine animale telles que le perhydrosqualène,
- les huiles végétales telles que l'huile d'amande douce, l'huile de calophyllum, l'huile de palme, l'huile d'avocat, l'huile de jojoba, l'huile d'olive, l'huile de ricin, les huiles de germes de ceréales telles que l'huile de germe de blé,
- les huiles de silicone telles que le diméthylpolysiloxane,
- les esters de synthèse tels que l'huile de purcelin, le myristate de butyle, le myristate d'isopropyle, le myristate de cétyle, le palmitate d'isopropyle, le stéarate de butyle, le stérate d'hexadécyle, le stéarate d'isopropyle, le stéarate d'octyle, le stéarate d'isocétyle, l'oléate de décyle, le laurate d'hexyle, le dicaprylate de propylène glycol, l'adipate de di-isopropyle,
- les alcools organiques tels que l'alcool oléique, l'alcool linoléique, l'alcool linolénique, l'alcool isostéarylique, l'octyl dodécanol,
- les esters dérivés d'acide lanolique tels que le lanolate d'isopropyle, le lanolate d'isocétyle,
- parmi les huiles on peut également citer : les acétylglycérides, les octanoates et décanoates d'alcools et de polyalcools tels que ceux de glycol et de glycérol, les ricinoléates d'alcools et de polyalcools tels que celui de cétyle.

On introduit également le plus souvent des cires. Parmi les cires susceptibles d'être utilisées selon l'invention, on peut mentionner :

- les cires minérales telles que les cires microcristallines, la paraffine, le pétrolatum, la vaseline,
- les cires fossiles telles que l'ozokérite, la cire de montan,
- les cires d'origine animale telles que la cire d'abeilles, le spermaceti, la cire de lanoline, les dérivés issus de la lanoline tels que les alcools de lanoline, la lanoline hydrogénée, la lanoline hydroxylée, la lanoline acétylée, les acides gras de la lanoline, l'alcool de lanoline acétylée,
- les cires d'origine végétale telles que la cire de candellila, la cire de Carnauba, la cire du Japon,
- les huiles hydrogénées concrètes à 25°C telles que l'huile de ricin hydrogénée, l'huile de palme hydrogénée, le suif hydrogéné, l'huile de coco hydrogénée,
- les esters gras concrets à 25°C tels que le monomyristate de propylène glycol, le myristate de myristyle,
- parmi les cires, on peut également citer : l'alcool cétylique, l'alcool stéarylique, les mono, di et triglycérides concrets à 25°C, le monoéthanolamide stéarique, la colophane et ses dérivés tels que les abiétates de glycol et de glycérol, les sucroglycérides et les oléates, myristates, lanolates, stéarates et dihydroxystéarates de calcium, magnésium, zinc et aluminium.

Des pigments sont introduits lorsque la composition cosmétique doit être colorée.

On ajoute également généralement des agents tensioactifs tels que les succinylglycérides, les alkylphosphates, les esters d'acide gras tels que les polysorbates vendus sous la dénomination "TWEEN" par la société ICI Americas, les esters de polyéthylèneglycol tels que ceux vendus sous la dénomination "BRIJ" par la société ICI.

La composition cosmétique anhydre selon l'invention est préparée par mélange des différents constituants. De préférence les éléments constituant la phase grasse et les éléments constituant la phase lipidique provésiculaire sont mélangés séparément, puis les deux phases obtenues sont mélangées jusqu'à obtenir une préparation homogène. Dans ce dernier cas les actifs cosmétiques et/ou dermopharmaceutiques sont de préférence introduits dans la phase lipidique provésiculaire.

Selon l'invention, la mise en contact de la composition cosmétique anhydre avec une phase aqueuse peut être effectuée juste avant application de la composition sur la peau. Dans ce but, on peut prélever la composition avec une éponge humide et étaler la composition sur la peau à l'aide de ladite éponge.

On peut également effectuer la mise en contact en étalant la composition sur la peau humectée au préalable soit par la salive ou la sueur, ou l'eau contenue dans les muqueuses, soit à l'aide d'une phase aqueuse provenant d'une source externe.

La mise en contact peut également être effectuée après application de la substance cosmétique anhydre. Dans ce cas également, la phase aqueuse peut être constituée par la sueur ou la salive ou provenir d'une source externe.

Les objectifs, les caractéristiques et les avantages de la présente invention apparaîtront plus clairement à la lecture des exemples ci-après donnés à titre illustratif et nullement limitatif.

Exemple 1 (comparatif)

1) Préparation d'une phase grasse A ayant la composition suivante :

| | |
|---|---|
| Polybutène | 5,04 g |
| Huile de lanoline | 20,38 g |
| Octoxyglycéryl Behenate | 20,38 g |
| Stéarylheptanoate | 9,84 g |
| Huile de jojoba | 9,84 g |
| Huile de ricin | 19,20 g |
| Butylhydroxytoluène | 0,06 g |
| Butylhydroxyanisole | 0,06 g |
| Cire Microcristalline | 7,60 g |
| Polyéthylène 500 | 7,60 g |

Le mélange des différents composés ci-dessus est effectué à une température comprise entre 100 et 120°C sous agitation au barreau aimanté jusqu'à obtention d'une préparation bien homogène.

2) On réalise ensuite à partir de cette phase grasse A les différentes compositions suivantes :

- Composition A1 (ne contenant pas de phase lipidique provésiculaire mais contenant un actif liposoluble) : On mélange 1% d'acétate d'alpha-tocophérol, qui est un composé actif liposoluble, à 99% de la phase grasse A définie ci-dessus.
- Composition A2 : (contenant une phase lipidique provésiculaire non-ionique mais pas d'actif liposoluble).

a) On prépare par cofusion à 100°C sous azote une phase lipidique provésiculaire ayant la composition suivante :

- lipide amphiphile non-ionique de formule :

$$C_{16}H_{33}O-\left[C_3H_5\ (OH)\ O\right]_{\bar{n}}-H \qquad (V)$$

formule dans laquelle :

$-C_3H_5$ (OH) O- est représenté par les structures suivantes, prises en mélange ou séparément :

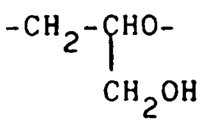 ; 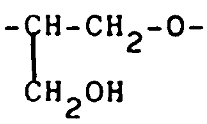 ;

et

| | |
|---|---|
| - $\bar{n}$ est une valeur statistique moyenne égale à 3 | 4,75 g |
| - cholestérol | 4,75 g |
| - dicétyl phosphate | 0,5 g |

b) La composition A2 est préparée en mélangeant 90% de phase grasse A et 10% de la phase lipidique provésiculaire décrite ci-dessus.

- Composition A3 (contenant une phase lipidique provésiculaire non-ionique et contenant un actif liposoluble).

  a) On prépare par cofusion à 100°C sous azote une phase lipidique provésiculaire ayant la composition suivante :

| | |
|---|---|
| - lipide amphiphile non-ionique de formule V | 4,75 g |
| - cholestérol | 4,75 g |
| - dicétyl phosphate | 0,5 g |
| - acétate d'alpha-tocophérol | 1 g |

  b) La composition A3 est préparée en mélangeant 89% de la phase grasse ci-dessus et 11% de phase lipidique provésiculaire.

  La composition A3 contient donc 1% d'acétate d'alphatocophérol.
- Composition A4 (contenant une phase lipidique provésiculaire ionique et ne contenant pas d'actif liposoluble).
  On prépare par le même procédé que pour la composition A3 une composition A4 constituée de :

  - 90% en poids de la phase grasse A
  - 10% d'une phase lipidique provésiculaire constituée de 6% de lécithine de soja vendue sous la dénomination commerciale "LECINOL S 1O" par la société NIKKO
  - 4 % de phytostérol oxyéthyléné à 5 modes d'oxyde d'éthylène vendu sous la dénomination "GENEROL 122 E5" par la société HENKEL.

- Composition A5 (contenant une phase lipidique provésiculaire ionique et un actif liposoluble).

  On prépare par le même procédé que pour la composition A1 la composition A5 constituée de :

- 89% de phase grasse A
- 11% d'une phase lipidique provésiculaire constituée de :

  . 6 % de "LECINOL S 1O"
  . 4 % de phytostérol oxyéthyléné à 5 moles d'oxyde d'éthylène vendu sous la dénomination "GENEROL 122 E5" par la société HENKEL et de
  . 1 % d'acétate d'alpha-tocophénol.

3) Essais comparatifs

Un film de 1 g de chacune des compositions A, A1, A2, A3, A4 et A5, est déposé dans une boîte de Pétri, puis recouvert d'une quantité de 10 g d'eau.

La boîte est fermée hermétiquement puis agitée sur une secoueuse pendant 24 h à la température ambiante.

Après 24 h, la phase aqueuse est récupérée et pesée. Une première partie aliquote de cette phase aqueuse obtenue est récupérée et observée au microscope à contraste de phase. Une seconde partie aliquote de cette phase

aqueuse est lyophilisée puis reprise dans un solvant (chloroforme). Le dosage des lipides vésiculaires (V) et du cholestérol ou du phytostérol est effectué par chromatographie en couche mince haute performance (CCMHP) après carbonisation, la lecture des plaques se faisant sur un densitomètre Shimadzu. Le dosage de l'acétate d'alpha-tocophérol est fait par chromatographie liquide sous haute pression (CLHP).

Les résultats sont donnés dans le tableau I ci-après :

Tableau I

| Formules | quantité de lipide (V)* | quantité de cholestérol ou de phytostérol* | Rapport pondéral (V)/Cholestérol ou phytostérol (%) | quantité d' Acétate d'α-tocophérol* en % | Résultat de l'examen au microscope |
|---|---|---|---|---|---|
| A** | 0 | 0 | - | 0 | Pas de vésicules |
| A1** | 0 | 0 | - | 0 | Pas de vésicules |
| A2 | 5% | 5% | 50/50 | 0 | Nombreux vésicules |
| A3 | 6% | 6% | 50/50 | 1,5 | Nombreux vésicules |
| A4 | 8% | 7% | 60/40 | 0 | vésicules |
| A5 | 8% | 6% | 60/40 | 3 | vésicules |

* dosée par rapport à la quantité initiale déposée dans la boîte de Pétri.
** ne fait pas partie de l'invention.

Les résultats donnés ci-dessus montrent que lorsqu'on ajoute à la phase grasse d'une composition cosmétique anhydre une phase lipidique provésiculaire, il est possible, par contact avec une phase aqueuse, de libérer dans cette phase aqueuse des vésicules ayant la même composition que la phase lipidique provésiculaire ajoutée et que, si l'on introduit dans la composition un actif liposoluble, ce dernier est également libéré dans la phase aqueuse avec les vésicules formées. Le simple mélange de l'actif liposoluble avec la phase grasse ne permet pas de libérer l'alpha-tocophérol dans la phase aqueuse.

Les techniques de dosage par chromatographie utilisées pour cet exemple sont les suivantes :

<u>Technique de dosage CCMHP</u>

Sur une plaque de gel de silice 60 Merck sans indicateur de fluorescence, on dépose 20 ul d'échantillon ou d'étalon.
La gamme d'étalonnage est réalisée de 0,5 mg/ml à 3 mg/ml.
La migration est faite dans une cuve verticale avec un mélange chloroforme/méthanol (80/20).
La révélation est faite par pulvérisation d'H SO à 20% dans l'eau puis carbonisation à 170°C pendant 5 min.
La lecture est faite sur un densitomètre Shimadzu à :

- 400 nm pour le cholestérol
- 470 nm pour le lipide amphiphile non-ionique de formule (V) et le phytostérol oxyéthyléné (GENEROL 122 E5)
- 530 nm pour la lécithine hydrogénée, commercialisée sous la dénomination "LECINOL S 1O" par la société NIKKO.

Technique de dosage par CLHP de l'acétate d'alpha-tocophérol.

| | |
|---|---|
| - Colonne | type RP 18 (5µm) qualité Lichrosorb commercialisée par MERCK |
| - Eluant | Méthanol pur |
| - Débit | 1,5 ml/min |
| - λ | 280 nm |
| - Injection | 10 µl |

Les étalons et échantillons sont en solution dans le chloroforme.

Exemple 2 (comparatif). Compositions préparées

à partir d'une phase grasse de rouge à lèvre.

1) Préparation des compositions

On prépare comme dans l'exemple 1 une base B ayant la composition suivante :

| | |
|---|---|
| Polybutylène | 5,04 g |
| Huile de lanoline | 20,38 g |
| Octoxyglycérylbéhénate | 20,38 g |
| Stéarylheptanoate | 9,84 g |
| Huile de jojoba | 9,84 g |
| Huile de ricin | 19,20 g |
| Butylhydroxytoluène | 0,06 g |
| Butylhydroxyanisole | 0,06 g |

à laquelle on ajoute pour obtenir des phases grasses des quantités variables, définies dans le tableau II ci-après, de cire de polyéthylène vendue sous la dénomination "POLYWAX 500" par la société BARECO et de cire microcristalline.

Aux phases grasses obtenues on a ajouté deux phases lipidiques provésiculaires.

La phase lipidique provésiculaire (1) a la composition suivantes :

| | |
|---|---|
| - lipide non-ionique de formule (V) décrit à l'exemple 1 | 47,5 g |
| - cholestérol | 47,5 g |
| - dicétylphosphate | 5,0 g |

La phase lipidique provésiculaire (2) a la composition suivante :

| | |
|---|---|
| - "LECINOL S 1O" | 60,0 g |
| - cholestérol | 30,0 g |
| - acide palmitoylcollagénique commercialisé par la société RHONE-POULENC sous la dénomination "LIPACIDE PCO" | 10,0 g |

Les deux phases lipidiques provésiculaires sont préparées par cofusion à 100°C sous azote.

On a également éventuellement ajouté par prémélange avec la phase lipidique provésiculaire, de la glycérine qui est un actif hydrosoluble.

On a obtenu des compositions B1 à B9 dont la composition est donnée dans le tableau II ci-après :

| Compositions | Base B en g | Cire microcristalline en g | Cire de Polyéthylène en g | Glycérine en g | Mélange poids pour poids de glycérine et de phase lipidique provésiculaire (1) en g | Mélange poids pour poids de glycérine et de phase lipidique provésiculaire (2) en g |
|---|---|---|---|---|---|---|
| | | | | | | |
| B1* | 78,0 | 8,50 | 8,50 | 5,0 | 0 | 0 |
| B2* | 72,8 | 8,60 | 8,60 | 10,0 | 0 | 0 |
| B3* | 67,7 | 8,65 | 8,65 | 15,0 | 0 | 0 |
| | | | | | | |
| B4 | 73,8 | 8,10 | 8,10 | | 10,0 | 0 |
| B5 | 65,5 | 7,25 | 7,25 | | 20,0 | 0 |
| B6 | 54,8 | 7,60 | 7,60 | | 30,0 | 0 |
| | | | | | | |
| B7 | 73,8 | 8,10 | 8,10 | | 0 | 10,0 |
| B8 | 65,5 | 7,25 | 7,25 | | 0 | 20,0 |
| B9 | 54,8 | 7,60 | 7,60 | | 0 | 30,0 |

* ne font pas partie de l'invention

2) Essais comparatifs

Un film de 1 g de chacune des phases grasses B1 à B9 est déposé dans une boîte de Pétri, puis recouvert d'une quantité de 10 g d'eau.

La boîte est fermée hermétiquement puis agitée sur une secoueuse pendant 24 h à la température ambiante.

Après 24 h, la phase aqueuse est récupérée et pesée.

Le dosage de la glycérine est effectué par voie enzymatique à l'aide du Kit Sigma 337 A.

Les résultats sont donnés dans le tableau III ci-après :

| Compositions | Quantité de glycérine dosée dans la phase aqueuse en % de la quantité initiale | Potentialisation (1) |
|---|---|---|
| | | |
| B1* | 8 | |
| B2* | 10 | |
| B3* | 7 | |
| | | |
| B4 | 43 | B4/B1: X 5,4 |
| B5 | 59 | B5/B2: X 5,9 |
| B6 | 86 | B6/B3: X 12,3 |
| | | |
| B7 | 20 | B7/B1: X 2,5 |
| B8 | 31 | B8/B2: X 3,1 |
| B9 | 42 | B9/B3: X 6 |

* ne font pas partie de l'invention

(1) La potentialisation est le facteur d'augmentation de la quantité d'actif libérée dans l'eau par rapport aux compositions ne contenant pas de phase lipidique provésiculaire.

Les résultats présentés dans cet exemple montrent que l'invention a permis de potentialiser la libération de la glycérine dans un environnement aqueux, quelles que soient la nature de la phase lipidique provésiculaire utilisée et la concentration en glycérine dans cette phase.

Le dosage de la glycérine selon le protocole Sigma du Kit n° 337 est effectué à l'aide du réactif défini ci-dessous selon les réactions :

$$\text{Glycérol} + \text{ATP} \xrightarrow{\text{GK}} \text{G-1-P} + \text{ADP}$$

$$\text{G-1-P} + O_2 \xrightarrow{\text{GPO}} \text{DAP} + H_2O_2$$

$$H_2O_2 + 4\ \text{AAP} + \text{ESPA} \xrightarrow{\text{POD}} \text{Quinonéimine colorée} + H_2O$$

| | |
|---|---|
| ATP | Adénosine Triphosphate |
| GK | Glycérol Kinase |
| G1P | Glycérol 1 phosphate |
| ADP | Adénosine 5' -diphosphate |
| GPO | Glycérol phosphate oxydase |
| DAP | Dihydroacétone phosphate |
| 4-AAP | 4 aminoantipyrine |
| ESPA | sodium N-éthyl-N(3-sulfopropyl)m-anisidine |
| POD | Peroxydase |

La lecture est faite à 540 nm.

Le réactif utilisé a la composition suivante :

| | |
|---|---|
| ATP | 0,375 mmol/l |
| Sel de Magnésium | 3,75 mmol/l |
| 4 aminoantipyrine | 0,188 mmol/l |
| Sodium N- éthyl-N[3-sulfopropyl] m-anisidine | 2,11 mmol/l |
| Glycérol kinase | 1.250 U/l |
| Glycérol phosphate oxydase | 2500 U/l |
| Peroxydase | 2500 U/l |
| Tampon | pH 7 |

A titre comparatif, on effectue le dosage d'un blanc et des différents échantillon dans les proportions suivantes :

| | Blanc | Echantillon |
|---|---|---|
| Réactif | 3 ml | 3 ml |
| Echantillon | - | 0,01 ml |
| Eau | 0,01 ml | - |

Le calcul de la concentration se fait par rapport à la densité optique d'un standard connu.

Exemple 3 (comparatif) : Fond de teint libérateur de liposomes ioniques chargés en glycérine ou en sorbitol.

1) Préparation.

On réalise la phase grasse C ayant la composition donnée ci-après par mélange des cires et des huiles à 80°C sous agitation jusqu'à obtention d'un mélange homogène, puis on ajoute les pigments et les charges toujours à 80°C et sous agitation jusqu'à obtention d'une couleur homogène ; on ajoute ensuite le reste des constituants et on maintient la température à 80°C pendant 2 heures sous agitation.
La phase grasse C préparée a la composition suivante :

| | |
|---|---|
| Cire microcristalline | 4 g |
| Cire de Carnauba | 6 g |
| Octyl palmitate | 14 g |
| Polyisobutène hydrogéné | 17,6 g |
| Trilaurine | 7 g |
| Propyl Paraben | 0,1 g |
| Oxyde de fer (jaune) | 4,9 g |
| Oxyde de fer (brun-jaune) | 1,9 g |
| Dioxyde de titane | 16,5 g |
| Oxyde de fer (noir) | 0,7 g |
| Oxyde de zinc | 3,0 g |
| Benzophénone-3 | 0,5 g |
| Octyl méthoxycinnamate | 0,5 g |
| Diméthicone vendue sous la dénomination "SILBIONE 70047 V300" par la société RHONE POULENC | 0,3 g |
| NYLON vendu sous la dénomination "L'ORGASOL 2002 Natural EXTRA COS" par la société ATO | 8,0 g |
| Mica | 15,0 g |

A partir de cette phase grasse C, on réalise, par mélange, en quantités variables, de glycérine ou de sorbitol avec une phase lipidique provésiculaire constituée d'un mélange de "LECINOL S 1O" et d'un phytostérol oxyéthyléné (GENEROL 122 E5), dans la proportion en poids de 60/40 à la température de 80°C sous agitation pendant 1 heure, les compositions suivantes:

| Compositions | Phase grasse C en g | Glycérine en g | Sorbitol en g | LECINOL S 1O/phytostérol 60/40 en g |
|---|---|---|---|---|
| C1 | 100 | - | - | - |
| C2 | 95 | 5 | - | - |
| C3 | 95 | - | - | 5 |
| C4 | 90 | 5 | - | 5 |
| C5 | 95 | - | 5 | - |
| C6 | 90 | - | 5 | 5 |

2) Essais

Ces compositions ont été testées suivant la méthode utilisée dans l'exemple 1 :

Mode opératoire

Un film de 1 g de chacune des bases $C_1$ à $C_6$ est déposé dans une boîte Pétri puis recouvert d'une quantité de 10 g d'eau.

La boîte est fermée hermétiquement puis agitée sur une secoueuse pendant 24 heures à la température ambiante. Après 24 heures, la phase aqueuse est récupérée puis quantifiée. Le dosage des lipides est fait par CCMHP, la glycérine est dosée par voie enzymatique comme décrit précédemment, et le sorbitol est dosé par voie enzymatique selon la méthode suivante.

Méthode de dosage du sorbitol

La méthode est basée sur la réaction suivante :

$$\text{D-Sorbitol} + NAD^+ \xrightarrow{SDH} \text{Fructose} + \text{NADH, H+}$$

(SDH = sorbitol deshydrogenase ; NAD = Nicotinamide Deshydrogenase) Dans les conditions de l'essai, la réaction est complètement déplacée vers la droite. La quantité de NADH,H+ formée dans la réaction est stoechiométrique avec la quantité de sorbitol ; elle est déterminée par mesure de l'augmentation d'absorbance à 340 nm.

Réactifs - préparation des solutions

Solution 1 : pyrophosphate 0,2M, pH = 9,5 Dissoudre 8,92 g de $Na_4P_2O_7$, 10 $H_2O$ dans 80 ml d'eau distillée Ajuster à pH 9,5 avec HC1 1 N puis au volume à 100 ml avec de l'eau distillée

Solution 2 : Dissoudre 40 mg de NAD dans 2 ml d'eau distillée

Solution 3 : Sorbitol Deshydrogénase (SDH) Dissoudre 2 mg dans 500 ul d'eau distillée

Mode opératoire :

- On prépare les solutions suivantes :

| | Blanc | Echantillon |
|---|---|---|
| Solution 1 | 1,00 ml | 1,00 ml |
| Solution 2 | 0,10 ml | 0,10 ml |
| Echantillon | - | 0,20 ml |
| Eau distillée | 1,70 ml | 1,50 ml |

On fait démarrer la réaction en ajoutant dans chaque cuve 0,05 ml de solution 3. On mélange. On lit les densités optiques après 60 min contre de l'eau distillée à 340 nm. La concentration en g/l de sorbitol est égale à 0,412 Δ Abs, - Δ Abs étant l'absorbance de l'échantillon moins l'absorbance du blanc.

Les résultats obtenus avec les compositions de fond de teint testées sont les suivants :

Tableau IV

| Formules | Quantité de lipides** | Rapport pondéral Lécinol S1O/phytostérol | Quantité d'actif dans la phase aqueuse** | Potentialisation |
|---|---|---|---|---|
| C1* | - | - | - | - |
| C2* | - | - | 12 | - |
| C3 | 7 | 60/40 | - | - |
| C4 | 6 | 60/40 | 78 | $C_4/C_2$: X 6,5 |
| C5* | - | | 8 | - |
| C6 | 6 | 60/40 | 49 | $C_6/C_5$: X 6,1 |

* ne fait pas partie de l'invention

** dosée par rapport à la quantité initiale (%)

Les résultats ci-dessus montrent qu'en présence d'une phase lipidique provésiculaire, des vésicules ayant la même composition que la phase lipidique sont libérées par simple contact avec un environnement aqueux. D'autre part, il est montré que les vésicules formées potentialisent la libération d'actifs hydrosolubles (glycérol ou sorbitol).

Exemple 4 : Préparation d'un "two way cake".

On prépare un liant L ayant la composition suivante :

| | |
|---|---|
| Huile de vaseline | 56,5 g |
| Huile de ricin | 10,8 g |
| Vaseline | 10,0 g |
| Myristate d'isopropyle | 7,2 g |
| Alcool oléique | 10,0 g |
| Lanoline | 5,5 g |

On prépare également par cofusion à 100°C sous azote une phase lipidique provésiculaire (3) ayant la composition suivante :

| | |
|---|---|
| - lipide amphiphile non-ionique de formule (V) de l'exemple 1 | 47,5 g |
| - cholestérol | 47,5 g |
| - dicétylphosphate | 5,0 g |

On mélange ensuite les charges, les pigments et les poudres jusqu'à obtenir un mélange homogène ; on ajoute ensuite le liant et la phase lipidique provésiculaire préalablement chauffés pour les amener à l'état liquide. On mélange puis on broie le tout afin d'obtenir une composition homogène.

Le "two way cake" obtenu a la composition suivante :

| | |
|---|---|
| Talc qsp | 100,0 g |
| Mica | 20,0 g |
| Oxyde de titane | 5,0 g |
| Stéarate de zinc | 2,0 g |
| Poudre de nylon | 5,0 g |
| Oxyde de fer | 2,0 g |
| Octyl diméthyl paraaminobenzoate | 0,5 g |
| Parfum | 0,2 g |
| Conservateur | 1,0 g |
| Liant L | 8,0 g |
| Phase lipidique provésiculaire (3) | 2,0 g |
| Glycérine | 2,0 g |
| Vitamine E | 0,1 g |

Il est appliqué par l'utilisatrice sur le visage à l'aide d'une éponge humide.

## Revendications

1. Procédé de traitement cosmétique selon lequel on applique sur la peau ou la muqueuse labiale une composition cosmétique anhydre de maquillage comprenant une phase grasse caractérisé par le fait que la composition cosmétique anhydre est préparée par mélange de la phase grasse avec une phase lipidique provésiculaire contenant au moins un lipide amphiphile susceptible de former des vésicules par contact avec une phase aqueuse et que la composition cosmétique est en contact avec une phase aqueuse, lorsqu'elle est appliquée.

2. Procédé selon la revendication 1, caractérisé par le fait que la mise en contact avec une phase aqueuse est effectuée juste avant l'application.

3. Procédé selon la revendication 1, caractérisé par le fait que la composition est appliquée sur la peau ou la muqueuse labiale humectée au préalable.

4. Procédé selon la revendication 1, caractérisé par le fait que la composition est mise en contact avec une phase aqueuse après application.

5. Procédé selon l'une des revendications 1 à 4, caractérisé par le fait que l'on introduit dans la composition cosmétique, au moins un actif cosmétique et/ou dermopharmaceutique hydrosoluble et/ou au moins un actif cosmétique et/ou dermopharmaceutique liposoluble.

6. Procédé selon l'une des revendications 1 à 5, caractérisé par le fait que l'on mélange la phase lipidique provésiculaire avec la phase grasse dans des proportions telles que la phase lipidique provésiculaire représente 0,1 à 30 % en poids de la composition cosmétique.

7. Procédé selon la revendication 6, caractérisé par le fait que l'on mélange la phase lipidique provésiculaire avec la phase grasse dans des proportions telles que la phase lipidique provésiculaire représente 1 à 20 % en poids de la composition cosmétique.

8. Procédé selon l'une des revendications 1 à 7, caractérisé par le fait que la phase lipidique provésiculaire contient au moins un lipide amphiphile ionique.

9. Procédé selon l'une des revendications 1 à 7, caractérisé par le fait que le lipide amphiphile ionique est choisi parmi les phospholipides naturels ou de synthèse.

10. Procédé selon la revendication 8, caractérisé par le fait que le lipide amphiphile ionique est au moins un lipide de formule (I) :

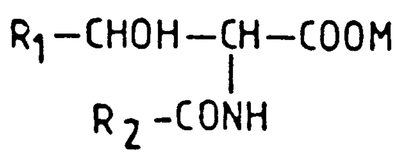

(I)

formule dans laquelle $R_1$ désigne un radical alkyle ou alcényle en $C_7$ - $C_{21}$ ; $R_2$ désigne un radical hydrocarboné, saturé ou insaturé en $C_7$ - $C_{31}$ ; et M représente H, Na, K, $NH_4$ ou un ion ammonium dérivé d'une amine et, notamment, d'une amine hydroxylée.

11. Procédé selon l'une des revendications 1 à 7, caractérisé par le fait que la phase lipidique provésiculaire contient au moins un lipide amphiphile non-ionique choisi dans le groupe formé par :

(1) Les dérivés de polyglycérol, linéaires ou ramifiés, de formule (II) :

$$R_3O-\left[C_3H_5(OH)O\right]_{\bar{n}}-H \qquad (II)$$

formule dans laquelle :
$-C_3H_5(OH)O-$ est représenté par les structures suivantes prises en mélange ou séparément : $-CH_2-CHOH-CH_2O-$ ;

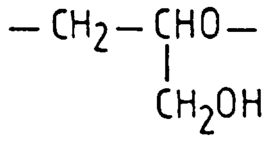 et 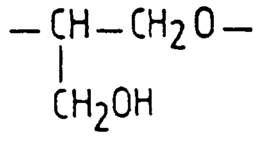

$\bar{n}$ est une valeur statistique moyenne comprise entre 1 et 6.
$R_3$ représente :

(a) une chaîne aliphatique, linéaire ou ramifiée, saturée ou insaturée, contenant de 12 à 30 atomes de carbone ; ou des radicaux hydrocarbonés des alcools de lanoline ;
(b) un reste $R_4$ CO, où $R_4$ est un radical aliphatique, linéaire ou ramifié, en $C_{11}$ - $C_{29}$ ;
(c) un reste

$$R_5\{OC_2H_3(R_6)\}$$

où $R_5$ peut prendre la signification (a) ou (b) donnée pour $R_3$ ;
$-OC_2H_3(R_6)-$ est représenté par les structures suivantes, prises en mélange ou séparément :

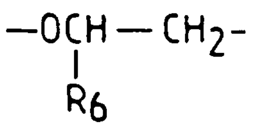 ; et 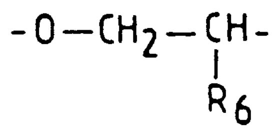

où $R_6$ prend la signification (a) donnée pour $R_3$ ;
(2) les éthers de polyglycérol, linéaires ou ramifiés, comportant deux chaînes grasses ;
(3) les alcools gras polyoxyéthylénés ;
(4) les stérols et phytostérols polyoxyéthylénés ;
(5) les éthers de polyols ;
(6) les esters de polyols oxyéthylénés ou non ;
(7) les glycolipides d'origine naturelle ou synthétique ;
(8) les alpha-diols polyglycérolés ;
(9) les hydroxyamides représentés par la formule :

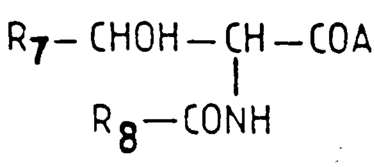

formule dans laquelle :

- $R_7$ représente un radical alkyle ou alcényle en $C_7$ - $C_{21}$ ;
- $R_8$ représente un radical hydrocarboné, saturé ou insaturé en $C_7$ - $C_{31}$ ;
- -COA représente un groupement choisi parmi les deux groupements suivants :

. un reste

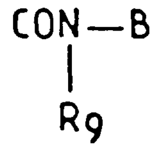

où :
B est radical alkyle dérivé d'amines primaires ou secondaires, mono ou polyhydroxylées ; et $R_9$ désigne un atome d'hydrogène ou un radical méthyle, éthyle ou hydroxyéthyle ; et
un reste -COOZ, où Z représente le reste d'un polyol en $C_3$ - $C_7$.

(10) les éthers et esters de formule (IV) :

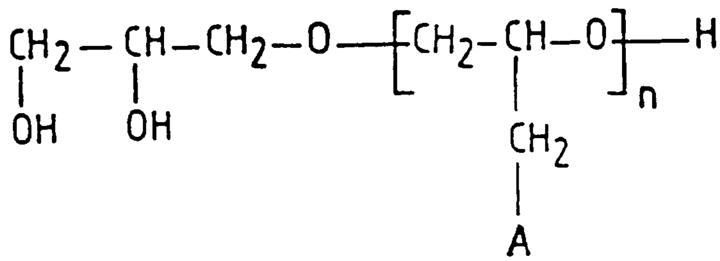

(IV)

formule dans laquelle A représente -OR ou

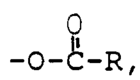

R représentant un radical hydrocarboné saturé ou insaturé et n représentant une valeur égale à 2 ou une valeur statistique moyenne $\bar{n}$ supérieure à 1 et au plus égale à 6.

**12.** Procédé selon l'une des revendications 1 à 7, caractérisé par le fait que la phase lipidique provésiculaire contient au moins un lipide amphiphile ionique et au coins un lipide amphiphile non-ionique.

**13.** Procédé selon l'une des revendications 1 à 12, caractérisé par le fait que la phase lipidique provésiculaire contient au moins un additif destiné à améliorer la stabilité et la perméabilité des vésicules.

**14.** Procédé selon la revendication 13, caractérisé par le fait que ledit additif est choisi parmi les alcools et diols à longue chaîne : les ammonium quaternaires ; les hydroxyalkylamines ; les amines grasses polyoxyéthylénées ; les esters d'aminoalcools à longue chaîne et leurs sels et dérivés ammonium quaternaires ; les esters phosphoriques d'alcools gras sous forme libre ou neutralisée et les alkyl-sulfates ; les dérivés ioniques des stérols et certains polymères.

**15.** Procédé selon la revendication 5, caractérisé par le fait que l'on introduit dans la composition cosmétique un actif cosmétique et/ou dermopharmaceutique liposoluble choisi dans le groupe formé par la vitamine E, les esters de vitamine E, les acides gras polyinsaturés, la vitamine F, les filtres solaires, les antioxydants, les conservateurs, la vitamine A, l'acide rétinoïque et ses esters.

**16.** Procédé selon la revendication 5, caractérisé par le fait que l'on introduit dans la composition cosmétique un actif cosmétique et/ou dermopharmaceutique hydrosoluble choisi dans le groupe formé par le glycérol, le sorbitol et autres polyols de structure proche, les acides aminés, les filtres solaires et les vitamines.

**17.** Utilisation d'une composition cosmétique anhydre préparée par mélange d'une phase grasse avec une phase lipidique amphiphile pour former des vésicules dont la paroi contient le(s) lipide(s) de ladite phase lipidique amphiphile, par simple contact avec une phase aqueuse sur la peau ou la muqueuse labiale.

**18.** Composition cosmétique anhydre de maquillage pour la mise en oeuvre du procédé selon l'une des revendications 1 à 4, caractérisée par le fait qu'elle est constituée par un mélange d'une phase grasse et d'un lipide amphiphile susceptible de former des vésicules choisi dans le groupe formé par :

(1) Les lipides amphiphiles ioniques suivants : la sphingomyéline et les phospholipides de synthèse,
(2) Les lipides anioniques représentés par la formule (I) :

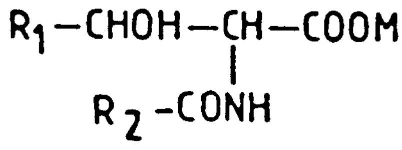

(I)

formule dans laquelle $R_1$ désigne un radical alkyle ou alcényle en $C_7$ - $C_{21}$ ; $R_2$ désigne un radical hydrocarboné, saturé ou insaturé en $C_7$ - $C_{31}$ ; et M représente H, Na, K, $NH_4$ ou un ion ammonium dérivé d'une amine et, notamment, d'une amine hydroxylée.
(3) Les lipides amphiphiles non-ioniques suivants :

(a) Les dérivés de polyglycérol, linéaires ou ramifiés, de formule (II) :

$$R_3O-[C_3H_5(OH)O]_{\bar{n}}-H \quad (II)$$

formule dans laquelle :
$-C_3H_5(OH)O-$ est représenté par les structures suivantes prises en mélange ou séparément : $-CH_2-CHOH-CH_2O-$ ;

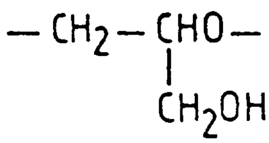 et 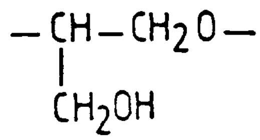

$\bar{n}$ est une valeur statistique moyenne comprise entre 1 et 6.
$R_3$ représente :

(α) une chaîne aliphatique, linéaire ou ramifiée, saturée ou insaturée, contenant de 12 à 30 atomes de carbone ; ou des radicaux hydrocarbonés des alcools de lanoline ;
(β) un reste $R_4$ CO, où $R_4$ est un radical aliphatique, linéaire ou ramifié, en $C_{11}$ - $C_{29}$ ;
(γ) un reste

$$R_5[OC_2H_3(R_6)]$$

où $R_5$ peut prendre la signification (a) ou (b) donnée pour $R_3$ ;
$-OC_2$ $H_3$ $(R_6)-$ est représenté par les structures suivantes, prises en mélange ou séparément :

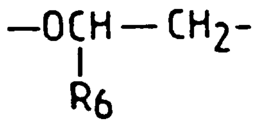 ; et 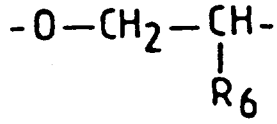

où $R_6$ prend la signification (a) donnée pour $R_3$ ;
(b) les éthers de polyglycérol, linéaires ou ramifiés, comportant deux chaînes grasses ;
(c) les alcools gras polyoxyéthylénés ;
(d) les stérols et phytostérols polyoxyéthylénés ;
(e) les éthers de polyols ;
(f) les esters de polyols oxyéthylénés ou non ;
(g) les glycolipides d'origine naturelle ou synthétique ;
(h) les alpha-diols polyglycérolés ;
(i) les hydroxyamides représentés par la formule :

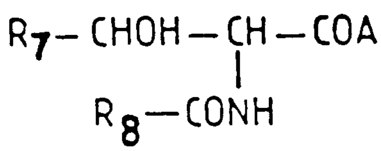

formule dans laquelle :

- $R_7$ représente un radical alkyle ou alcényle en $C_7$-$C_{21}$ ;
- $R_8$ représente un radical hydrocarboné, saturé ou insaturé en $C_7$ - $C_{31}$ ;
- -COA représente un groupement choisi parmi les deux groupements suivants :

. un reste

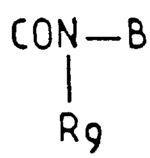

où :
B est radical alkyle dérivé d'amines primaires ou secondaires, mono ou polyhydroxylées ; et $R_9$ désigne un atome d'hydrogène ou un radical méthyle, éthyle ou hydroxyéthyle ; et

. un reste -COOZ, où Z représente le reste d'un polyol en $C_3$ - $C_7$.

(j) les éthers et esters de formule (IV) :

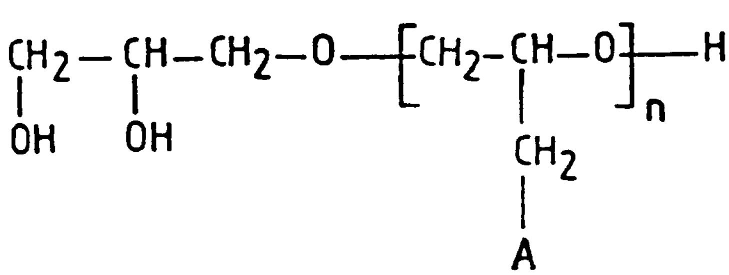 (IV)

formule dans laquelle A représente -OR ou

$$-O-\overset{O}{\overset{\|}{C}}-R,$$

R représentant un radical hydrocarboné saturé ou insaturé et n représentant une valeur égale à 2 ou une valeur statistique moyenne $\overline{n}$ supérieure à 1 et au plus égale à 6 ;

et les mélanges de deux de ces composés ou plus.

**19.** Composition selon la revendication 18, caractérisée par le fait qu'elle contient au moins un actif cosmétique et/ou dermopharmaceutique hydrosoluble et/ou au moins un actif cosmétique et/ou dermopharmaceutique liposoluble.

**20.** Composition selon l'une des revendications 18 ou 19, caractérisée par le fait que la phase lipidique provésiculaire représente 0,1 à 30 % en poids de la composition cosmétique.

**21.** Composition selon la revendication 20, caractérisée par le fait que la phase lipidique provésiculaire représente 1 à 20 % en poids de la composition cosmétique.

**22.** Composition selon l'une des revendications 18 à 21, caractérisée par le fait que la phase lipidique provésiculaire contient au moins un lipide amphiphile ionique et au moins un lipide amphiphile non-ionique.

**23.** Composition selon la revendication 18, caractérisée par le fait que la phase lipidique provésiculaire contient au moins un additif destiné à améliorer la stabilité et la perméabilité des vésicules.

**24.** Composition selon la revendication 23, caractérisée par le fait que ledit additif est choisi parmi les alcools et diols à longue chaîne ; les ammonium quaternaires ; les hydroxyalkylamines ; les amines grasses polyoxyéthylénées ; les esters d'aminoalcools à longue chaîne et leurs sels et dérivés ammonium quaternaires ; les esters phosphoriques d'alcools gras sous forme libre ou neutralisée et les alkyl-sulfates ; les dérivés ioniques des stérols et certains polymères.

**25.** Composition selon la revendication 19, caractérisée par le fait que l'on introduit dans la composition cosmétique un actif cosmétique et/ou dermopharmaceutique liposoluble choisi dans le groupe formé par la vitamine E, les esters de vitamine E, les acides gras polyinsaturés, la vitamine F, les filtres solaires, les antioxydants, les conservateurs, la vitamine A, l'acide rétinoïque et ses esters.

**26.** Composition selon la revendication 19, caractérisée par le fait que l'on introduit dans la composition cosmétique un actif cosmétique et/ou dermopharmaceutique hydrosoluble choisi dans le groupe formé par le glycérol, le sorbitol et autres polyols de structure proche, les acides aminés, les filtres solaires et les vitamines.

**Claims**

**1.** Cosmetic treatment process according to which an anhydrous cosmetic make-up composition comprising a fatty phase is applied to the skin or the labial mucosa, characterized in that the anhydrous cosmetic composition is prepared by mixing the fatty phase with a provesicular lipid phase containing at least one amphiphilic lipid capable of forming vesicles by contact with an aqueous phase, and in that the cosmetic composition is in contact with an aqueous phase when it is applied.

**2.** Process according to Claim 1, characterized in that the contacting with an aqueous phase is performed immediately before application.

**3.** Process according to Claim 1, characterized in that the composition is applied to the skin or the labial mucosa moistened beforehand.

**4.** Process according to Claim 1, characterized in that the composition is brought into contact with an aqueous phase after application.

5. Process according to one of Claims 1 to 4, characterized in that at least one water-soluble cosmetic and/or dermopharmaceutical active agent and/or at least one fat-soluble cosmetic and/or dermopharmaceutical active agent is/are introduced into the cosmetic composition.

6. Process according to one of Claims 1 to 5, characterized in that the provesicular lipid phase is mixed with the fatty Phase in proportions such that the provesicular lipid phase represents 0.1 to 30 % by weight of the cosmetic composition.

7. Process according to Claim 6, characterized in that the provesicular lipid phase is mixed with the fatty phase in proportions such that the provesicular lipid phase represents 1 to 20 % by weight of the cosmetic composition.

8. Process according to one of Claims 1 to 7, characterized in that the provesicular lipid phase contains at least one ionic amphiphilic lipid.

9. Process according to one of Claims 1 to 7, characterized in that the ionic amphiphilic lipid is chosen from natural or synthetic phospholipids.

10. Process according to Claim 8, characterized in that the ionic amphiphilic lipid is at least one lipid of formula (I):

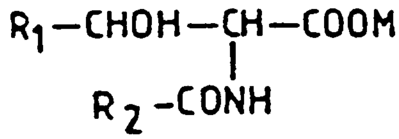 (I)

in which formula $R_1$ denotes a $C_7$-$C_{21}$ alkyl or alkenyl radical; $R_2$ denotes a saturated or unsaturated $C_7$-$C_{31}$ hydrocarbon radical; and M represents H, Na, K, $NH_4$ or an ammonium ion derived from an amine, and in particular from a hydroxylated amine.

11. Process according to one of Claims 1 to 7, characterized in that the provesicular lipid phase contains at least one nonionic amphiphilic lipid chosen from the group composed of:

(1) the linear or branched polyglycerol derivatives of formula (II):

$$R_3O-\left[C_3H_5(OH)O\right]_{\bar{n}}-H \qquad (II)$$

in which formula:
$-C_3H_5(OH)O-$ is represented by the following structures, taken mixed or separately: $-CH_2-CHOH-CH_2O-$ ;

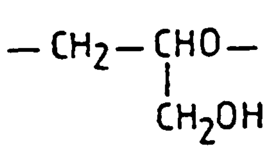 and 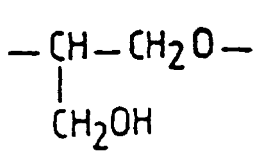

$\bar{n}$ is an average statistical value between 1 and 6; $R_3$ represents:

(a) a saturated or unsaturated, linear or branched aliphatic chain containing from 12 to 30 carbon atoms; or hydrocarbon radicals of lanolin alcohols;
(b) a residue $R_4CO$, where $R_4$ is a linear or branched $C_{11}$-$C_{29}$ aliphatic radical;
(c) a residue

$$R_5[OC_2H_3(R_6)]$$

where $R_5$ can take the meaning (a) or (b) given for $R_3$;
$-OC_2H_3(R_6)-$ is represented by the following structures, taken mixed or separately:

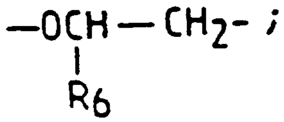

and

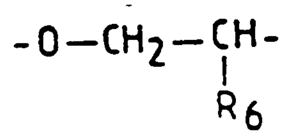

where $R_6$ takes the meaning (a) given for $R_3$;

(2) linear or branched polyglycerol ethers containing two fatty chains;
(3) polyoxyethylenated fatty alcohols;
(4) polyoxyethylenated sterols and phytosterols;
(5) polyol ethers;
(6) polyol esters, oxyethylenated or otherwise;
(7) glycolipids of natural or synthetic origin;
(8) polyglycerolated alpha-diols;
(9) the hydroxyamides represented by the formula:

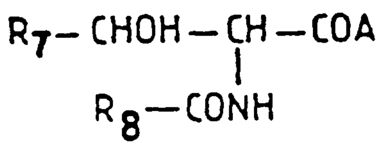

in which formula:

- $R_7$ represents a $C_7$-$C_{21}$ alkyl or alkenyl radical;
- $R_8$ represents a saturated or unsaturated $C_7$-$C_{31}$ hydrocarbon radical;
- -COA represents a group chosen from the following two groups:
  - a residue

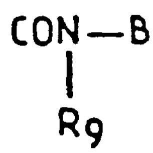

    where:
    B is an alkyl radical derived from mono- or polyhydroxylated primary or secondary amines; and $R_9$ denotes a hydrogen atom or a methyl, ethyl or hydroxyethyl radical; and
  - a residue -COOZ, where Z represents the residue of a $C_3$-$C_7$ polyol.

(10) the ethers and esters of formula (IV):

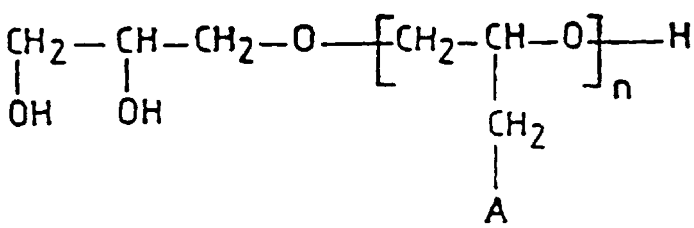

(IV)

in which formula A represents -OR or

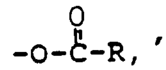

R representing a saturated or unsaturated hydrocarbon radical, and n representing a value equal to 2 or an average statistical value $\bar{n}$ greater than 1 and not exceeding 6.

**12.** Process according to one of Claims 1 to 7, characterized in that the provesicular lipid phase contains at least one ionic amphiphilic lipid and at least one nonionic amphiphilic lipid.

**13.** Process according to one of Claims 1 to 12, characterized in that the provesicular lipid phase contains at least one additive intended for improving the stability and permeability of the vesicles.

**14.** Process according to Claim 13, characterized in that the said additive is chosen from long-chain alcohols and diols; quaternary ammonium compounds; hydroxyalkylamines; polyoxyethylenated fatty amines; esters of long-chain amino alcohols and their salts and quaternary ammonium derivatives; phosphoric esters of fatty alcohols in free or neutralized form, and alkyl sulphates; ionic derivatives of sterols; and certain polymers.

**15.** Process according to Claim 5, characterized in that a fat-soluble cosmetic and/or dermopharmaceutical active agent, chosen from the group composed of vitamin E, vitamin E esters, polyunsaturated fatty acids, vitamin F, sunscreen agents, antioxidants, preservatives, vitamin A, retinoic acid and its esters, is introduced into the cosmetic composition.

**16.** Process according to Claim 5, characterized in that a water-soluble cosmetic and/or dermopharmaceutical active agent, chosen from the group composed of glycerol, sorbitol and other polyols of related structure, amino acids, sunscreen agents and vitamins, is introduced into the cosmetic composition.

**17.** Use of an anhydrous cosmetic composition prepared by mixing a fatty phase with an amphiphilic lipid phase to form vesicles whose wall contains the lipid(s) of the said amphiphilic lipid phase, by simple contact with an aqueous phase on the skin or the labial mucosa.

**18.** Anhydrous cosmetic make-up composition for carrying out the process according to one of Claims 1 to 4, characterized in that it consists of a mixture of a fatty phase and an amphiphilic lipid capable of forming vesicles, chosen from the group composed of:

(1) The following ionic amphiphilic lipids: sphingomyelin and synthetic phospholipids;
(2) The anionic lipids represented by the formula (I):

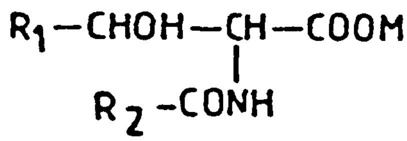
(I)

in which formula $R_1$ denotes a $C_7$-$C_{21}$ alkyl or alkenyl radical; $R_2$ denotes a saturated or unsaturated $C_7$-$C_{31}$ hydrocarbon radical; and M represents H, Na, K, $NH_4$ or an ammonium ion derived from an amine, and in particular from a hydroxylated amine;
(3) The following nonionic amphiphilic lipids:

(a) the linear or branched polyglycerol derivatives of formula (II):

$$R_3O-\left[C_3H_5(OH)O\right]_{\bar{n}}-H \qquad (II)$$

in which formula:

$-C_3H_5(OH)O-$ is represented by the following structures, taken mixed or separately: $-CH_2-CHOH-CH_2O-$ ;

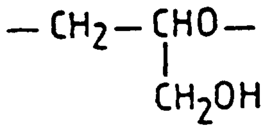

and

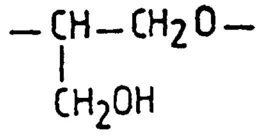

$\bar{n}$ is an average statistical value between 1 and 6;

$R_3$ represents:

(α) a saturated or unsaturated, linear or branched aliphatic chain containing from 12 to 30 carbon atoms; or hydrocarbon radicals of lanolin alcohols;

(β) a residue $R_4CO$, where $R_4$ is a linear or branched $C_{11}$-$C_{29}$ aliphatic radical;

(γ) a residue

$$R_5[OC_2H_3(R_6)]$$

where $R_5$ can take the meaning (α) or (β) given for $R_3$;

$-OC_2H_3(R_6)-$ is represented by the following structures, taken mixed or separately:

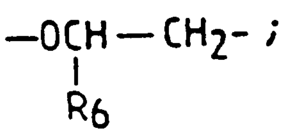

and

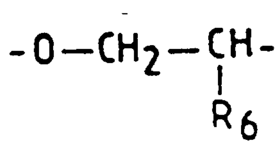

where $R_6$ takes the meaning (α) given for $R_3$;

(b) linear or branched polyglycerol ethers containing two fatty chains;

(c) polyoxyethylenated fatty alcohols;

(d) polyoxyethylenated sterols and phytosterols;

(e) polyol ethers;

(f) polyol esters, oxyethylenated or otherwise;

(g) glycolipids of natural or synthetic origin;

(h) polyglycerolated alpha-diols;

(i) the hydroxyamides represented by the formula:

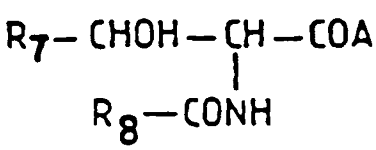

in which formula:

- $R_7$ represents a $C_7$-$C_{21}$ alkyl or alkenyl radical;
- $R_8$ represents a saturated or unsaturated $C_7$-$C_{31}$ hydrocarbon radical;
- -COA represents a group chosen from the following two groups:

- a residue

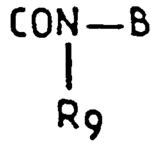

where:
B is an alkyl radical derived from mono- or polyhydroxylated primary or secondary amines; and
$R_9$ denotes a hydrogen atom or a methyl, ethyl or hydroxyethyl radical; and

- a residue -COOZ, where Z represents the residue of a $C_3$-$C_7$ polyol.

(j) the ethers and esters of formula (IV):

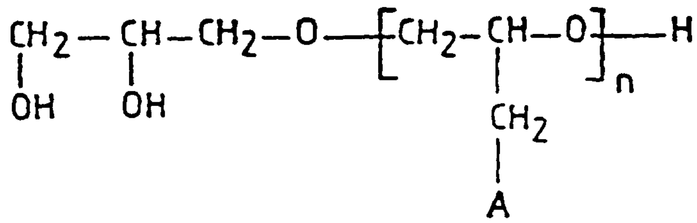

(IV)

in which formula A represents -OR or

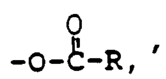

R representing a saturated or unsaturated hydrocarbon radical, and n representing a value equal to 2 or an average statistical value $\bar{n}$ greater than 1 and not exceeding 6;

and mixtures of two or more of these compounds.

**19.** Composition according to Claim 18, characterized in that it contains at least one water-soluble cosmetic and/or dermopharmaceutical active agent and/or at least one fat-soluble cosmetic and/or dermopharmaceutical active agent.

**20.** Composition according to either of Claims 18 and 19, characterized in that the provesicular lipid phase represents 0.1 to 30 % by weight of the cosmetic composition.

**21.** Composition according to Claim 20, characterized in that the provesicular lipid phase represents 1 to 20 % by weight of the cosmetic composition.

**22.** Composition according to one of Claims 18 to 21, characterized in that the provesicular lipid phase contains at least one ionic amphiphilic lipid and at least one nonionic amphiphilic lipid.

**23.** Composition according to Claim 18, characterized in that the provesicular lipid phase contains at least one additive intended for improving the stability and permeability of the vesicles.

**24.** Composition according to Claim 23, characterized in that the said additive is chosen from long-chain alcohols and diols; quaternary ammonium compounds; hydroxyalkylamines; polyoxyethylenated fatty amines; esters of long-chain amino alcohols and their salts and quaternary ammonium derivatives; phosphoric esters of fatty alcohols in free or neutralized form, and alkyl sulphates; ionic derivatives of sterols; and certain polymers.

25. Composition according to Claim 19, characterized in that a fat-soluble cosmetic and/or dermopharmaceutical active agent, chosen from the group composed of vitamin E, vitamin E esters, polyunsaturated fatty acids, vitamin F, sunscreen agents, antioxidants, preservatives, vitamin A, retinoic acid and its esters, is introduced into the cosmetic composition.

26. Composition according to Claim 19, characterized in that a water-soluble cosmetic and/or dermopharmaceutical active agent, chosen from the group composed of glycerol, sorbitol and other polyols of related structure, amino acids, sunscreen agents and vitamins, is introduced into the cosmetic composition.

## Patentansprüche

1. Kosmetisches Behandlungsverfahren, nach dem man eine wasserfreie, kosmetische Schminke, die eine Fettphase enthält, auf die Haut oder Lippenschleimhaut aufträgt, dadurch gekennzeichnet, daß die wasserfreie kosmetische Zusammensetzung durch Mischen der Fettphase mit einer provesikulären Lipidphase, die mindestens ein amphiphiles Lipid enthält, das bei Kontakt mit einer wässrigen Phase Vesikeln zu bilden vermag, hergestellt wird, und daß die kosmetische Zusammensetzung beim Auftragen mit einer wässrigen Phase in Kontakt ist.

2. Verfahren gemäß Anspruch 1, dadurch gekennzeichnet, daß das In-Kontakt-Bringen mit einer wässrigen Phase unmittelbar vor dem Auftragen erfolgt.

3. Verfahren gemäß Anspruch 1, dadurch gekennzeichnet, daß die Zusammensetzung auf die Haut oder die zuvor angefeuchtete Lippenschleimnaut aufgetragen wird.

4. Verfahren gemäß Anspruch 1, dadurch gekennzeichnet, daß die Zusammensetzung nach dem Auftragen mit einer wässrigen Phase in Kontakt gebracht wird.

5. Verfahren gemäß einem der Ansprüche 1 bis 4, dadurch gekennzeichnet, daß man in die kosmetische Zusammensetzung mindestens einen wasserlöslichen kosmetischen und/oder dermatopharmazeutischen Wirkstoff und/oder mindestens einen fettlöslichen kosmetischen und/oder dermatopharmazeutischen Wirkstoff einbringt.

6. Verfahren gemäß einem der Ansprüche 1 bis 5, dadurch gekennzeichnet, daß man die provesikuläre Lipidphase mit der Fettphase in einem solchen Verhältnis mischt, daß die provesikuläre Lipidphase 0,1 bis 30 Gew.-% der kosmetischen Zusammensetzung ausmacht.

7. Verfahren gemäß Anspruch 6, dadurch gekennzeichnet, daß man die provesikuläre Lipidphase mit der Fettphase in einem solchen Verhältnis mischt, daß die provesikuläre Lipidphase 1 bis 20 Gew.-% der kosmetischen Zusammensetzung ausmacht.

8. Verfahren gemäß einem der Ansprüche 1 bis 7, dadurch gekennzeichnet, daß die provesikuläre Lipidphase mindestens ein amphiphiles, ionisches Lipid enthält.

9. Verfahren gemäß einem der Ansprüche 1 bis 7, dadurch gekennzeichnet, daß das amphiphile, ionische Lipid aus natürlichen oder synthetischen Phospholipiden ausgewählt wird.

10. Verfahren gemäß Anspruch 8, dadurch gekennzeichnet, daß das amphiphile, ionische Lipid mindestens ein Lipid der Formel (I) ist:

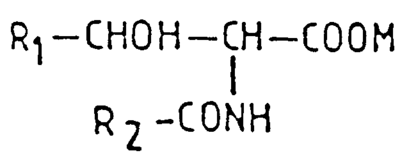

(I)

worin
$R_1$ ein ($C_7$-$C_{21}$)-Alkyl- oder -Alkenylrest ist;
$R_2$ ein gesättigter oder ungesättigter ($C_7$-$C_{31}$)-Kohlenwasserstoffrest ist, und
M ein H, Na, K, $NH_4$ oder ein Ammoniumionderivat eines Amins und insbesondere eines Hydroxylamins ist.

**11.** Verfahren gemäß einem der Ansprüche 1 bis 7, dadurch gekennzeichnet, daß die provesikuläre Lipidphase mindestens ein amphiphiles, nicht-ionisches Lipid enthält, das aus der Gruppe der folgenden Verbindungen gewählt wird:

(1) linearen oder verzweigten Polyglycerinderivaten der Formel (II):

$$R_3O-[C_3H_5(OH)O]_{\bar{n}}-H \qquad (II)$$

worin
$-C_3H_5(OH)O-$ für jede der folgenden Strukturen in beliebiger Kombination steht:
$-CH_2-CHOH-CH_2O-$ ;

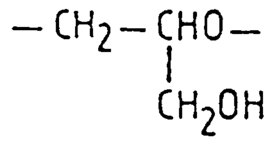 und 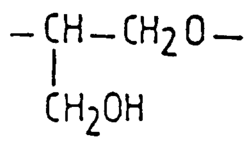

$\bar{n}$ ein statistischer Mittelwert zwischen 1 und 6 ist. $R_3$ für:

(a) eine lineare oder verzweigte, gesättigte oder ungesättigte aliphatische Kette, die 12 bis 30 Kohlenstoffatome enthält, oder für Kohlenwasserstoffreste von Lanolinalkoholen;
(b) einen Rest $R_4CO$, worin $R_4$ einen linearen oder verzweigten aliphatischen $(C_{11}-C_{29})$-Rest darstellt;
(c) einen Rest

$$R_5[OC_2H_3(R_6)]$$

worin $R_5$ dieselbe Bedeutung wie unter (a) oder (b) für $R_3$ angegeben, haben kann;
$-OC_2H_3(R_6)-$ für jede der nachfolgenden Strukturen in beliebiger Kombination steht:

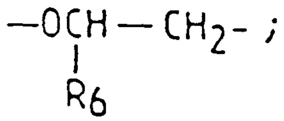 und 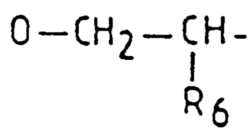

worin $R_6$ die für $R_3$ angegebene Bedeutung (a) hat;
2) linearen oder verzweigten Polyglycerinethern, die zwei Fettketten tragen;
(3) polyethoxylierten Fettalkoholen;
(4) polyethoxylierten Sterolen und Phytosterolen;
(5) Polyolethern;
(6) Polyolestern, ethoxyliert oder nicht;
(7) Glycolipiden natürlichen oder synthetischen Ursprungs;
(8) α-Diolpolyglycerinen;

(9) Hydroxyamiden der Formel

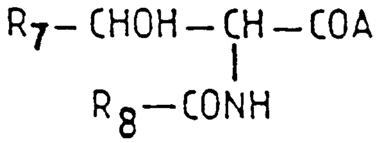

worin

- $R_7$ für einen ($C_7$-$C_{21}$)-Alkyl- oder -Alkenylrest steht;
- $R_8$ für einen gesättigten oder ungesättigten ($C_7$-$C_{31}$)-Kohlenwasserstoffrest steht;
- -COA für eine Gruppe, die aus den beiden folgenden Gruppen gewählt wird, steht:

  - einen Rest

    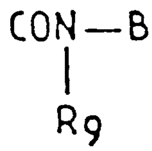

    worin:
    B einen von einem primären oder sekundären, mono- oder polyhydroxylierten Amin abgeleiteten Alkylrest steht;
    $R_9$ ein Wasserstoffatom oder ein Methyl-, Ethyl- oder Hydroxyethylrest ist;
  - und einen Rest -COOZ, worin Z ein ($C_3$-$C_7$) Polyolrest ist.

(10) Ethern und Estern der Formel (IV):

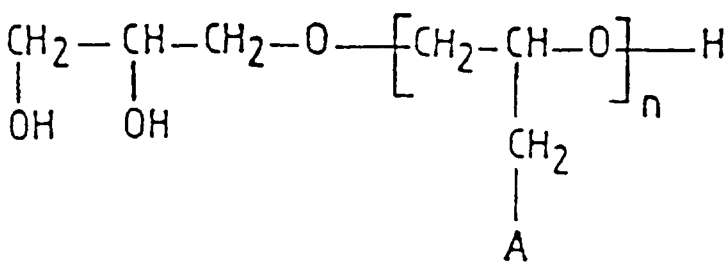

(IV)

worin
A für -OR oder

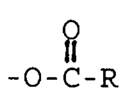

steht,
R für einen gesättigten oder ungesättigten Kohlenwasserstoffrest steht, und
n ein Wert von 2 oder ein statistischer Mittelwert $\bar{n}$ größer als 1 und höchstens gleich 6 ist.

**12.** Verfahren gemäß einem der Ansprüche 1 bis 7, dadurch gekennzeichnet, daß die provesikuläre Lipidphase mindestens ein amphiphiles ionisches Lipid und mindestens ein amphiphiles nicht-ionisches Lipid enthält.

13. Verfahren gemäß einem der Ansprüche 1 bis 12, dadurch gekennzeichnet, daß die provesikuläre Lipidphase mindestens ein Additiv zur Verbesserung der Stabilität und Durchlässigkeit der Vesikeln enthält.

14. Verfahren gemäß Anspruch 13, dadurch gekennzeichnet, daß das Additiv aus langkettigen Alkoholen und Diolen; quaternären Ammoniumverbindungen; Hydroxyalkylaminen; polyethoxylierten Fettaminen; Estern langkettiger Aminoalkohole und deren Salzen und quaternären Ammoniumderivaten; Phosphorsäureestern von Fettalkoholen in ihrer freien Form oder neutralisiert und den Alkylsulfaten; ionischen Derivaten von Sterolen und gewissen Polymeren ausgewählt wird.

15. Verfahren gemäß Anspruch 5, dadurch gekennzeichnet, daß man in die kosmetische Zusammensetzung einen fettlöslichen kosmetischen und/oder dermatopharmazeutischen Wirkstoff einbringt, der aus der Gruppe der folgenden Verbindungen gewählt wird: Vitamin E, Estern des Vitamin E, mehrfach ungesättigten Fettsäuren, Vitamin F, Sonnenfiltern, Antioxidantien, Konservierungsmitteln, Vitamin A, Retinoesäure und deren Estern.

16. Verfahren gemäß Anspruch 5, dadurch gekennzeichnet, daß man in die kosmetische Zusammensetzung mindestens einen wasserlöslichen kosmetischen und/oder dermatopharmazeutischen Wirkstoff einbringt, der aus der Gruppe der folgenden Verbindungen gewählt wird: Glycerin, Sorbit und anderen Polyolen verwandter Struktur, Aminosäuren, Sonnenfiltern und Vitaminen.

17. Verwendung einer wasserfreien kosmetischen Zusammensetzung, hergestellt durch Mischen einer Fettphase mit einer amphiphilen Lipidphase, so daß Vesikeln gebildet werden, deren Wandung das/die Lipid(e) der amphiphilen Lipidphase enthält, durch einfachen Kontakt mit einer wässrigen Phase auf der Haut oder der Lippenschleimhaut.

18. Wasserfreie, kosmetische Schminke zur Durchführung eines Verfahrens gemäß einem der Ansprüche 1 bis 4, dadurch gekennzeichnet, daß sie besteht aus einer Mischung einer Fettphase und einem amphiphilen Lipid, das Vesikeln zu bilden vermag, das aus der Gruppe der folgenden Verbindungen gewählt wird:

(1) den nachfolgenden amphiphilen ionischen Lipiden: Sphingomyelin und synthetischen Phospholipiden,
(2) den anionischen Lipiden der Formel (I):

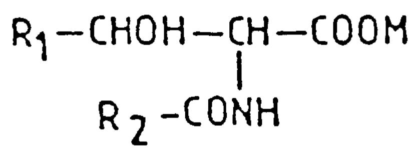

(I)

worin
$R_1$ ein $(C_7\text{-}C_{21})$-Alkyl- oder -Alkenylrest ist;
$R_2$ ein gesättigter oder ungesättigter $(C_7\text{-}C_{31})$-Kohlenwasserstoffrest ist; und
M ein H, Na, K, $NH_4$ oder ein Ammoniumionderivat eines Amins und insbesondere eines Hydroxylamins ist;
(3) den nachfolgenden amphiphilen, nicht-ionischen Lipiden:

(a) linearen oder verzweigten Polyglycerinderivaten der Formel (II):

$$R_3O-\left[C_3H_5(OH)O\right]_{\bar{n}}-H \qquad (II)$$

worin
$-C_3H_5(OH)O-$ für jede der folgenden Strukturen in beliebiger Kombination steht:
$-CH_2\text{-}CHOH\text{-}CH_2O-$ ;

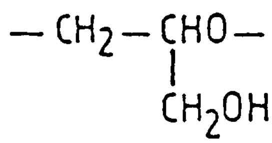 und 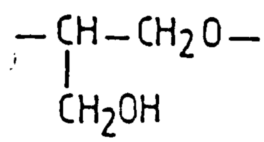

$\overline{n}$ ein statistischer Mittelwert zwischen 1 und 6 ist.

$R_3$ für:

(α) eine lineare oder verzweigte, gesättigte oder ungesättigte aliphatische Kette, die 12 bis 30 Kohlenstoffatome enthält oder für Kohlenwasserstoffreste von Lanolinalkoholen;

(β) einen Rest $R_4CO$, worin $R_4$ einen linearen oder verzweigten aliphatisches ($C_{11}$-$C_{29}$)-Rest darstellt;

(γ) einen Rest

$$R_5[OC_2H_3(R_6)]$$

worin

$R_5$ dieselbe Bedeutung wie unter (a) oder (b) für $R_3$ angegeben haben kann;

-$OC_2H_3(R_6)$- für jede der nachfolgenden Strukturen in beliebiger Kombination steht:

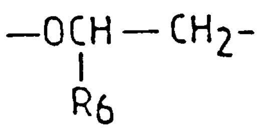 ; und 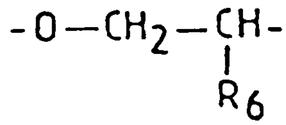

worin $R_6$ die für $R_3$ angegebene Bedeutung (a) hat;

(b) linearen oder verzweigten Polyglycerinethern, die zwei Fettketten tragen;

(c) polyethoxylierten Fettalkoholen;

(d) polyethoxylierten Sterolen und Phytosterolen;

(e) Polyolethern;

(f) Polyolestern, ethoxyliert oder nicht;

(g) Glycolipiden natürlichen oder synthetischen Ursprungs;

(h) α-Diolpolyglycerinen;

(i) Hydroxyamiden der Formel:

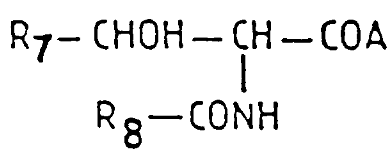

worin:

- $R_7$ für einen ($C_7$-$C_{21}$)-Alkyl- oder -Alkenylrest steht;
- $R_8$ für einen gesättigten oder ungesättigten ($C_7$-$C_{31}$)-Kohlenwasserstoffrest steht;
- -COA für eine Gruppe, die aus den beiden folgenden Gruppen gewählt wird, steht:

- einen Rest

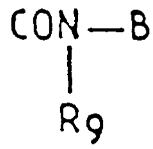

worin:
B einen von einem primären oder sekundären, mono- oder polyhydroxylierten Amin abgeleiteten Alkylrest steht;
und
$R_9$ ein Wasserstoffatom oder ein Methyl-, Ethyl- oder Hydroxyethylrest ist; und

- einen Rest -COOZ, worin Z ein ($C_3$-$C_7$)-Polyolrest ist.

(10) Ethern und Estern der Formel (IV):

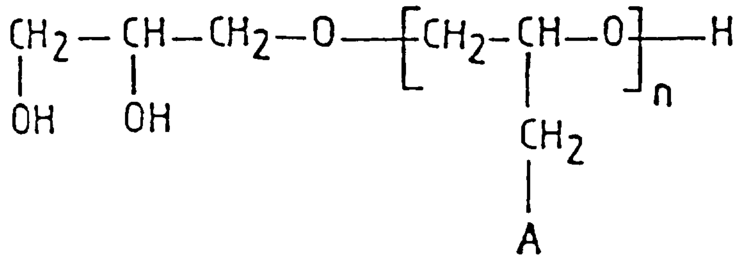
(IV)

worin
A für -OR oder

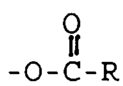

steht,
R für einen gesättigten oder ungesättigten Kohlenwasserstoffrest steht, und
n ein Wert von 2 oder ein statistischer Mittelwert $\bar{n}$ größer als 1 und höchstens gleich 6 ist

und den Mischungen von zwei oder mehreren dieser Verbindungen.

**19.** Zusammensetzung gemäß Anspruch 18, dadurch gekennzeichnet, daß sie mindestens einen wasserlöslichen kosmetischen und/oder dermatopharmazeutischen Wirkstoff und/oder mindestens einen fettlöslichen kosmetischen und/oder dermatopharmazeutischen Wirkstoff enthält.

**20.** Zusammensetzung gemäß einem der Ansprüche 18 oder 19, dadurch gekennzeichnet, daß die provesikuläre Lipidphase 0,1 bis 30 Gew.-% der kosmetischen Zusammensetzung ausmacht.

**21.** Zusammensetzung gemäß Anspruch 20, dadurch gekennzeichnet, daß die provesikuläre Lipidphase 1 bis 20 Gew.-% der kosmetischen Zusammensetzung ausmacht.

**22.** Zusammensetzung gemäß einem der Ansprüche 18 bis 21, dadurch gekennzeichnet, daß die provesikuläre Lipidphase mindestens ein amphiphiles, ionisches Lipid und mindestens ein amphiphiles nicht-ionisches Lipid enthält.

23. Zusammensetzung gemäß Anspruch 18, dadurch gekennzeichnet, daß die provesikuläre Lipidphase mindestens ein Additiv zur Verbesserung der Stabilität und Durchlässigkeit der Vesikeln enthält.

24. Zusammensetzung gemäß Anspruch 23, dadurch gekennzeichnet, daß das Additiv aus langkettigen Alkoholen und Diolen; quaternären Ammoniumverbindungen; Hydroxyalkylaminen; polyethoxylierten Fettaminen; Estern langkettiger Aminoalkohole und deren Salzen und quaternären Ammoniumderivaten; Phosphorsäureestern von Fettalkoholen in der freien Form oder neutralisiert oder den Alkylsulfaten; ionischen Derivaten von Sterolen und bestimmten Polymeren ausgewählt wird.

25. Zusammensetzung gemäß Anspruch 19, dadurch gekennzeichnet, daß man in die kosmetische Zusammensetzung einen fettlöslichen kosmetischen und/oder dermatopharmazeutischen Wirkstoff einbringt, der aus der Gruppe der folgenden Verbindungen gewählt wird: Vitamin E, Estern des Vitamin E, mehrfach ungesättigten Fettsäuren, Vitamin F, Sonnenfiltern, Antioxidantien, Konservierungsmitteln, Vitamin A, Retinoesäure und deren Estern.

26. Zusammensetzung gemäß Anspruch 19, dadurch gekennzeichnet, daß man in die kosmetische Zusammensetzung mindestens einen wasserlöslichen kosmetischen und/oder dermatopharmazeutischen Wirkstoff einbringt, der aus der Gruppe der folgenden Verbindungen gewählt wird: Glycerin, Sorbit und anderen Polyolen verwandter Struktur, Aminosäuren, Sonnenfiltern und Vitaminen.